# EUROPÄISCHE PATENTSCHRIFT

(11) **EP 0 851 741 B1**
(45) Veröffentlichungstag und Bekanntmachung des Hinweises auf die Patenterteilung: **23.08.2006**
(21) Anmeldenummer: 96938947.7
(22) Anmeldetag: 20.09.1996
(51) Int. Cl.: A61B 17/225

(54) **VORRICHTUNG ZUR BEHANDLUNG VON KÖRPERGEWEBE UND ZUR ZERTRÜMMERUNG VON KÖRPERSTEINEN**
DEVICE FOR TREATING BODY TISSUE AND CRUSHING CALCULI
DISPOSITIF PERMETTANT DE TRAITER DES TISSUS ORGANIQUES ET DE DETRUIRE DES CALCULS

(30) Priorität: 20.09.1995 DE 19534809
(43) Veröffentlichungstag der Anmeldung: 08.07.1998
(73) Patentinhaber: STORZ MEDICAL AG, CH-8280 Kreuzlingen (CH)
(72) Erfinder: WESS, Othmar, CH-8574 Lengwil-Oberhofen (CH)
(74) Vertreter: Lohr, Georg
(86) Internationale Anmeldenummer: PCT/DE1996/001794
(87) Internationale Veröffentlichungsnummer: WO 1997/010758

(56) Entgegenhaltungen:
- EP-A- 0 131 654
- EP-A- 0 240 797
- WO-A-93/08749
- DE-A- 2 913 251
- DE-A- 3 817 094
- DE-A- 4 122 590

## Beschreibung

### Technisches Gebiet

Die Erfindung bezieht sich auf eine Vorrichtung zur Behandlung von Körpergewebe und/oder zur Zertrümmerung von Körpersteinen mittels akustischer Energie, also beispielsweise mittels Stoß-, Druck- oder Ultraschallwellen gemäß dem Oberbegriff des Patentanspruchs 1.

### Stand der Technik

Eine Vorrichtung, von der bei der Formulierung des Oberbegriffs des Patentanspruchs 1 ausgegangen wird, ist aus der EP-A-0 369 177 bekannt. In dieser Druckschrift, auf die sowie den in dieser Druckschrift einschließlich des Recherchenberichts genannten Stand der Technik zur Erläuterung aller hier nicht oder nicht im einzelnen beschriebenen Begriffe ausdrücklich verwiesen wird, ist eine Vorrichtung beschrieben, die u.a. als extrakorporaler Lithotripter eingesetzt werden kann.

Die bekannten Vorrichtungen dieser Art weisen eine Schallerzeugungseinheit auf, die ein ebenes, ein zylindrisches, ein angenähert kugelförmiges oder ein bereits fokussiertes Wellenfeld in einem Übertragungsmedium erzeugt. Sofern das erzeugte Wellenfeld nicht die gewünschte "Bündelung" bzw. Fokussierung aufweist, ist der Schallerzeugungseinheit eine Bündelungseinheit für die akustischen Wellen nachgeordnet. Die Bündelungseinheit kann ein Reflektor mit einem - je nach Ausbildung der Schallerzeugungseinheit - parabolischen oder elliptischen Querschnitt, ein Stoßwellenrohr und/oder eine akustische Linse usw. sein.

Bei den bekannten Verfahren der extrakorporal induzierten Lithotripsie oder der Ultraschallthermotherapie sind die Schallerzeugungseinheit und die gegebenenfalls vorgesehene Bündelungseinheit außerhalb des menschlichen Körpers, d.h. extrakorporal angeordnet. Je nach durchzuführendem Behandlungsvorgang werden kontinuierliche, gepulste oder repetitive akustische Wellenfelder (Druck-, Stoß-, gepulste oder ungepulste Ultraschallfelder) außerhalb des Körpers erzeugt, über eine Einkoppelfläche in den Körper eingekoppelt und im Körper am Zielort konzentriert.

Bei den derzeit kommerziell erhältlichen Lithotriptoren werden die akustischen Wellen in Wasser erzeugt und dann über ein Wasserkissen oder auch im offenen Wasserbad in den Körper eingekoppelt. Um eine gute Einkopplung zu gewährleisten, bietet sich scheinbar Wasser als Transmissionsmedium an, weil man in guter Näherung davon ausgehen kann, daß Körpergewebe und Wasser eine ähnliche akustische Impedanz haben. Dadurch wird die akustische Energie ohne große Reflexionsverluste in den Körper übertragen. Wasser als Transmissionsmedium bietet sich auch wegen der einfachen Handhabbarkeit an. Dies ist der Grund dafür, daß heute alle bekannten Lithotriptoren und Ultraschallthermotherapiegeräte mit Wasser als Übertragungsmedium arbeiten.

Dabei ist bislang von folgendem ausgegangen worden:

Die akustische Impedanz Z ist definiert durch das Produkt von Dichte und der Schallausbreitungsgeschwindigkeit c. Sofern die Impedanz Z des Übertragungsmediums gleich oder zumindest angenähert gleich der Impedanz des Körpergewebes ist, ist eine verlustarme Energieeinkopplung gewährleistet. Dieses Ziel wird auch dann erreicht, wenn sich die Werte für die Dichte und Schallgeschwindigkeit in den beiden Medien deutlich unterscheiden, solange nur das Produkt der jeweiligen Werte für beide Medien zumindest angenähert gleich ist.

### Darstellung der Erfindung

Die Erfindung geht dagegen von folgender Erkenntnis aus:

Für die therapeutische Wirkung der Druck-, Stoß- oder Ultraschallwellen ist nicht allein die Menge der eingekoppelten Energie von Bedeutung, sondern die Konzentration der Energie auf den eigentlichen Wirkungsbereich. Die im Körper erreichbare fokale Energiedichte wird ganz entscheidend davon beeinflußt, ob die Wellen sich im Körper ungestört ausbreiten können, oder ob sie durch Hindernisse von der vorgesehenen Ausbreitungsrichtung abgelenkt, d.h. gebrochen, gestreut oder gebeugt werden. Dabei sind die Abweichungen von der gewünschten Ausbreitungsrichtung umso größer, je weiter sie vor der Wirkungszone erfolgen.

Hindernisse, an denen Abweichungen von der geradlinigen Ausbreitung erfolgen, sind z.B. Organgrenzen, an denen durch eine Änderung der Ausbreitungsgeschwindigkeit Brechung und Reflexion stattfinden. Beispielsweise unterscheidet sich die Ausbreitungsgeschwindigkeit von Ultraschallwellen in der Niere oder in der Leber teilweise um mehr als 50 m/s von der in Wasser.

Erfindungsgemäß ist nun erkannt worden, daß dieser Effekt z.B. in der Lithotripsie nicht zu vernachlässigen ist.

Dieser Effekt führt nämlich zu einer lateralen Aufweitung der Fokus- bzw. Therapiezone, die - je nach Patient und Steinlage -etwa ± 5 mm betragen kann. Eine derart große Aufweitung der Fokuszone führt zu einer signifikanten Reduktion der Energiedichte im Therapiefokus. Damit wird, wie im Vergleich mit Laborversuchen nachgewiesen ist, auch die gewünschte therapeutische Wirkung - hier der Zertrümmerungserfolg - deutlich verringert.

Zwar ist es nur durch aufwendige Anpassungen an die individuelle Anatomie und Ausbreitungsverhältnisse möglich ist, diese Abweichungen völlig zu kompensieren, erfindungsgemäß ist aber erkannt worden, daß bereits die erste Grenzfläche zwischen dem Medium, in dem die akustischen Wellen erzeugt werden, und dem Körpergewebe einen entscheidenden Einfluß auf die erreichbaren Energiedichten im Fokus hat:

Die Eintrittsfläche der akustischen Wellen, d.h. die Haut des Patienten, hat deshalb einen besonderen Einfluß, da sie am weitesten von der Fokuszone im Inneren des Körpers entfernt ist, sich also geringe Abweichungen von der geradlinigen Ausbreitung auf dem Weg zur Fokuszone in erheblichen lateralen Abweichungen auswirken. Darüberhinaus hat das unmittelbar an die Haut angrenzende Fettgewebe eine deutlich von Wasser unterschiedliche Ausbreitungsgeschwindigkeit für akustische Wellen, so daß große Brechungseffekte gerade an dieser Grenzfläche zu erwarten sind:

Die Ausbreitungsgeschwindigkeit im Fettgewebe beträgt zwischen 1350 und 1480 m/s, während sie in Wasser bei Temperaturen zwischen ca. 20°C und 50°C zwischen 1480 m/s und 1550 m/s liegt.

Der Erfindung liegt deshalb die Aufgabe zugrunde, die Effizienz bzw. den Wirkungsgrad von Therapiegeräten, die mit konzentrierten akustischen Wellen arbeiten, dadurch zu erhöhen, daß Abweichungen von der geradlinigen oder vorgesehenen Ausbreitung von akustischen Wellen beim Übergang vom Übertragungsmedium in den Körper minimiert und dadurch die Energiedichte im Fokus gesteigert wird.

Eine erfindungsgemäße Lösung dieser Aufgabe ist im Patentanspruch 1 angegeben. Weiterbildungen der Erfindung sind Gegenstand der Ansprüche 2 folgende.

Erfindungsgemäß wird die Form der Grenzfläche bzw. die Bedingungen an der Grenzfläche zwischen Übertragungsmedium und Körper derart eingestellt, daß die Übertragung der Energie von der Schallerzeugungseinheit in den Körper ohne wesentliche Abweichungen von der gewünschten Ausbreitung erfolgt,

Die Einstellung der Bedingungen an der Grenzfläche kann beispielsweise dadurch erfolgen, daß als Medium für die Übertragung von akustischen Wellen ein Material verwendet wird, das bezüglich des Brechungsindex, oder anders ausgedrückt der Ausbreitungsgeschwindigkeit, möglichst genau den oberflächlichen Gewebeschichten des menschlichen Körpers angepaßt ist, also ein Index-Matching ausgeführt wird:

Während man davon ausgehen kann, daß das oberflächennahe Fettgewebe eine Ausbreitungsgeschwindigkeit für akustische Wellen von etwa 1350-1400 m/s besitzt, muß man bei Wasser von 32°C mit etwa 1520 m/s, bei höheren Temperaturen mit noch höheren Werten rechnen. Zusätzlich soll das bisher benutzte Kriterium der Impedanzanpassung so gut wie möglich erfüllt werden, um unnötige Reflexionsverluste zu vermeiden. Primär aber muß darauf geachtet werden, daß die Brechungsindizes möglichst gut übereinstimmen, also keine Brechung der akustischen Wellen beim Übergang in den Körper erfolgt, durch die die Fokuszone lateral im Vergleich mit dem "regulären" Übertritt der Schallwellen aufgeweitet wird.

Medien, die die Bedingung des "Index-Matching" in guter Näherung erfüllen, sind z.B. gewisse Öle, die mit Ausbreitungsgeschwindig-keiten von z.B. 1400 m/s erhältlich sind. Als Beispiel sei hier Paraffinöl genannt. Je nach Anwendungsbereich und anatomischen Verhältnissen können andere Öle, weitere Flüssigkeiten oder auch Festkörper - insbesondere als "Anpaßschicht" - verwendet werden. Auch Mischungen derartiger Stoffe können Verwendung finden. Bei der Verwendung von Suspensionen ist darauf zu achten, daß die suspendierten Teilchen die Schallwellen nicht streuen.

Dabei ist es weitgehend unerheblich, ob die akustischen Wellen bereits in einem Medium mit angepaßtem Brechungsindex erzeugt werden, oder ob nur die Schicht des Transmissionsmediums, die mit dem Patientenkörper in Berührung kommt, angepaßt ist.

Unerheblich ist ebenfalls, ob das Transmissionsmedium in unmittelbarem Kontakt mit dem Körper steht oder ob ein oder mehrere dünne Grenzschichten, wie Koppelkissen, Zwischenfolien o.ä. einen direkten Kontakt verhindern. Solange diese Zwischenschichten vergleichsweise dünn sind, wird auch bei schrägem Durchgang der akustischen Wellen durch diese Schichten keine wesentliche Ablenkung von der geradlinigen Ausbreitung auftreten.

Eine weitere - alternative oder zusätzliche - Möglichkeit, unerwünschte Abweichungen von der geradlinigen oder vorgesehenen Ausbreitung der akustischen Wellen zu vermeiden, besteht darin, die Geometrie der Grenzfläche so zu formen, daß Abweichungen nicht oder nur in kontrollierter Weise erfolgen:

Dies kann z.B. anders als bei den heute gebräuchlichen Lithotriptoren und Geräten zur Ultraschallthermotherapie dadurch erfolgen, daß die Ankoppelfläche kontrolliert verändert wird. Z.B. lassen sich durch Andruck entsprechend geformter Applikatoren ebene, sphärische oder anderweitig regelmäßig bzw. kontrolliert geformte Grenzflächen erzeugen, deren Wirkungen auf die Ausbreitung der akustischen Wellen gezielt berücksichtigt werden können. Auch damit lassen sich Wirkungszonen mit hoher Energiedichte im Körperinneren erzeugen und unerwünschte Abweichungen von der idealen Ausbreitung weitgehend vermeiden.

In jedem Falle erhält man durch die Erfindung eine Vorrichtung, deren Effizienz gegenüber herkömmliche Therapiegeräten, die mit konzentrierten akustischen Wellen arbeiten, gesteigert ist. Diese Effizienzsteigerung ist eine Folge der erfindungsgemäßen Maßnahmen, durch die Abweichungen bei der Brechung der Wellen vom "idealen Durchgang" beim Übergang der akustischen Wellen vom Übertragungsmedium in den Patientenkörper minimiert werden. Dadurch läßt sich ein kleinerer Fokus als bei herkömmlichen Geräten und damit eine erhöhte Energiedichte im Wirkungsbereich erreichen.

### Kurze Beschreibung der Zeichnung

Die Erfindung wird nachstehend anhand eines Ausführungsbeispiels unter Bezugnahme auf die Zeichnung näher erläutert, in der zeigen:
- Fig. 1: einen Querschnitt durch eine erfindungsgemäße Vorrichtung, und
- Fig. 2a bis 2c: schematisierte Darstellungen zur Erläuterung der Erfindung.

### Darstellung eines Ausführungsbeispiels

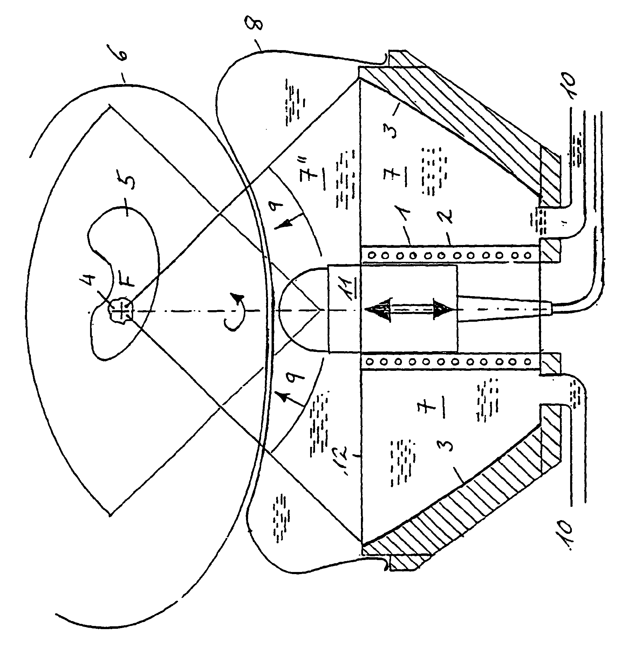

Fig. 1 zeigt ohne Beschränkung als Beispiel eine Vorrichtung zur Behandlung von Körpergewebe und/oder zur Zertrümmerung von Körpersteinen mittels Druckwellen, wie sie in der EP-A-0 369 177 beschrieben ist. Selbstverständlich kann die Erfindung auch bei anderen Vorrichtungen angewandt werden, wie sie beispielsweise in der genannten Druckschrift als Stand der Technik gewürdigt sind, und die Schallquellen anderer Konfiguration aufweisen.

Die in Fig. 1 dargestellte Vorrichtung weist als Schallquelle bzw. als Schallerzeugungseinheit eine Zylinderspule 1, die von einer Membran 2 umgeben ist. Die Schallquelle strahlt ein zylindrisches Wellenfeld ab, das ein Reflektor 3 "idealerweise in den kleinen Bereich F fokussiert. Der Reflektor 3 hat die Form eines Rotationsparaboloids. Am "Fokuspunkt" F befindet sich ein Stein 4 in einer Niere 5. Mit 6 ist die Außenkontur eines menschlichen Körpers bezeichnet.

Bei dem gezeigten Ausführungsbeispiel ist der Reflektor 3 von einer Folie bzw. einer Membran 8 abgeschlossen, so daß sich eine geschlossene Kammer 7 ergibt, die mit einem Übertragungsmedium für die von der Schallerzeugungseinheit erzeugten akustischen Wellen gefüllt ist. Das Übertragungsmedium leitet die akustischen Wellen, das die Wellen 9 zu dem Körper 6. Mit 10 sind Leitungen bezeichnet, mit denen das Übertragungsmedium in die Kammer 7 gepumpt bzw. auf dieser abgepumpt werden kann. 11 bezeichnet eine U1-traschall-Ortungseinheit, die innerhalb der Schallquelle angeordnet und in Richtung der Achse verschiebbar ist.

Soweit ist die in Fig. 1 dargestellte Vorrichtung aus der EP-A-0 369 177 bekannt. Bei der bekannten Vorrichtung ist das Übertragungsmedium, das sich in der Kammer 7 befindet, Wasser, das einen anderen Brechungsindex als das Körpergewebe hat.

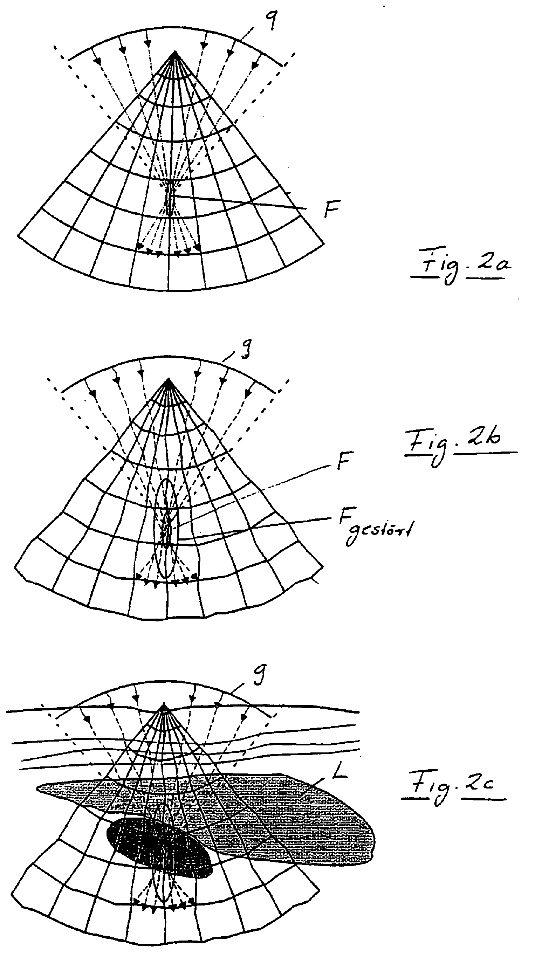

In Fig. 2 werden die gleichen Bezugszeichen wie in Fig. 1 verwendet, so daß auf eine erneute Vorstellung verzichtet wird.

Fig. 2a zeigt die Verhältnisse, die sich bei ungestörter Wellenausbreitung ergeben. Das "gerade" Gitter symbolisiert die Ausbreitung der Ultraschallwellen. Wie man aus Fig. 2a sieht, werden die Ultraschallwellen in einem Bereich F mit kleiner lateraler Erstreckung fokussiert.

Fig. 2b zeigt die Verhältnisse, die sich bei einer Wellenausbreitung ergeben, die durch die fehlende Index-Anpassung gestört wird. Das "wellenförmige" Gitter symbolisiert die gestörte Ausbreitung der Ultraschallwellen. Wie man aus Fig. 2b sieht, werden die Ultraschallwellen in einem Bereich gestört mit großer lateraler Erstreckung fokussiert.

Erfindungsgemäß können diese Störung zumindest verringert dadurch werden, daß das Übertragungsmedium in der Kammer 7 im Brechungsindex oder, mit anderen Worten, mit seiner Ausbreitungsgeschwindigkeit an die oberflächlichen Gewebeschichten des Körpers angepaßt wird. Hierzu wird als Übertragungsmedium ein Fluid gewählt, das eine Ausbreitungsgeschwindigkeit für akustische Wellen im Bereich von 1350 - 1400 m/s aufweist. Ein geeignetes Fluid ist beispielsweise ein ein Öl und insbesondere ein Paraffinöl.

Eine zweite Möglichkeit besteht darin, daß die Geometrie der Einkoppelfläche kontrolliert gestaltet wird, z.B. zu einer ebenen, sphärischen oder asphärischen Fläche verformt wird, deren Einfluß auf die Ausbreitung der akustischen Wellen bekannt ist und bei der Ermittelung der Fokuslage berücksichtigt wird. Hierzu wird die Folie bzw. die Membran 8, die bei den Koppelkissen herkömmlicher Vorrichtungen verwendet wird, durch einen Applikator ersetzt, der mit vergleichsweise hohem Druck an den Körper angedrückt wird, so daß sich eine definierte Grenzflächengeometrie zwischen dem menschlichen Körper 6 und der Schallaustrittsfläche der Vorrichtung ergibt. Beim Stand der Technik ergibt sich jedoch eine von der Körperkontur abhängige Grenzflächengeometrie.

Verwendet man nichtrotationssymmetrische Grenzflächen, so lassen sich auch Fokusverbreiterungen vermeiden, wie sie durch die Streuung an Organen, wie beispielsweise der Leber ergeben. Dies ist in Fig. 2c schematisch dargestellt, die die realen Bedingungen im menschlichen Körper mit Niere und Leber L zeigt.

Alternativ ist es auch möglich, die Kammer 7 beispielsweise bei der Linie durch eine Membran mit definierter Form oder gezielt änderbarer Form in zwei Kammern zu teilen, von denen die Kammer, die die Schallquelle 1 umgibt, mit Wasser und die Kammer 7", die an den Körper 8 angrenzt, mit einem Fluid mit angepaßtem Brechungsindex zu füllen.

In jedem Falle ist es mit der erfindungsgemäßen Vorrichtung zur Behandlung von Körpergewebe und zur Zertrümmerung von Körperkonkrementen möglich, hohe Energiedichten von Druck-, Stoß-oder Ultraschallwellen im Inneren des Körpers zu erzeugen. Unabhängig von der Art der angestrebten Wirkung in der Wirkungszone der Vorrichtung wird mit Hilfe der erfindungsgemäßen Vorrichtung die Wirkung der eingebrachten akustischen Energie in der definierten Wirkungs- oder Fokuszone dadurch erhöht, daß die Konzentration der Energie auf diese Zone gegenüber herkömmlichen Verfahren verbessert wird, also eine intensivere Wirkung in der vorbestimmten Zone und gleichzeitig eine Schonung des umliegenden Gewebes erreicht wird.

## Patentansprüche

1. Vorrichtung zur Behandlung von Körpergewebe und/oder zur Zertrümmerung von Körpersteinen (4) mittels akustischer Energie, wie Stoß-, Druck- oder Ultraschallwellen, mit
- einer Schallerzeugungseinheit (1, 2), die akustische Wellen erzeugt,
- gegebenenfalls einer nachgeordneten Bündelungseinheit (3) für die akustischen Wellen, und
- einem Übertragungsmedium für die von der Schallerzeugungseinheit erzeugten akustischen Wellen, das diese zu dem Körper leitet,
**dadurch gekennzeichnet, dass** der Brechungsindex bzw. die Ausbreitungsgeschwindigkeit des Übertragungsmediums an das oberflächliche Körpergewebe angepasst ist, so dass keine Brechung der akustischen Wellen beim Übergang in den Körper erfolgt.

2. Vorrichtung nach Anspruch 1,
**dadurch gekennzeichnet, dass** das Übertragungsmedium eine Ausbreitungsgeschwindigkeit für akustische Wellen im Bereich von 1350-1400 m/s aufweist.

3. Vorrichtung nach einem der Ansprüche 1 oder 2,
**dadurch gekennzeichnet, dass** das Übertragungsmedium ein Öl ist.

4. Vorrichtung nach Anspruch 3,
**dadurch gekennzeichnet, dass** das Übertragungsmedium Paraffinöl ist.

5. Vorrichtung nach einem der Ansprüche 1 bis 4,
**dadurch gekennzeichnet, dass** ein geschlossenes Behältnis (7) für das Übertragungsmedium vorgesehen ist, das eine von einem schalldurchlässigen Material (8) gebildete Schallaustrittsfläche aufweist, die die Grenzfläche bzw. die Koppelfläche bildet.

6. Vorrichtung nach Anspruch 5,
**dadurch gekennzeichnet, dass** das Behältnis (7) zwei Kammern aufweist, die durch eine definierte Schallübertrittsfläche getrennt sind, und von denen in der inneren Kammer die Schallerzeugungseinheit (1, 2) und die gegebenenfalls vorgesehene Bündelungseinheit (3) für die akustischen Wellen vorgesehen ist, und die äußere Kammer von der Schallaustrittsfläche (8) abgeschlossen ist, die die Grenzfläche zum Körper bildet.

7. Vorrichtung nach Anspruch 6,
**dadurch gekennzeichnet, dass** sich lediglich in der äußeren Kammer ein Medium mit angepasster Ausbreitungsgeschwindigkeit befindet.

8. Vorrichtung nach Anspruch 7,
**dadurch gekennzeichnet, dass** sich in der inneren Kammer Wasser befindet.

9. Vorrichtung nach einem der Ansprüche 5 bis 8,
**dadurch gekennzeichnet, dass** die Form der Schallübertrittsfläche geändert werden kann.

10. Vorrichtung nach einem der Ansprüche 5 bis 9,
**dadurch gekennzeichnet, dass** die Schallaustrittsfläche (8) eine Membran ist.

11. Vorrichtung zur Behandlung von Körpergewebe nach Anspruch 1,
**dadurch gekennzeichnet, dass** ein Applikator vorgesehen ist, welcher den Reflektor (3) abschliesst und derart ausgebildet ist, dass er unter vergleichsweise hohem Druck an den Körper angedrückt, die Geometrie der Einkoppelfläche in den Körper kontrolliert gestaltet und z. B. zu einer ebenen, asphärischen oder sphärischen Fläche verformt.

12. Vorrichtung nach einem der Ansprüche 1 bis 11,
**dadurch gekennzeichnet, dass** zusätzlich eine Impedanzanspassung erfolgt.

13. Vorrichtung nach einem der Ansprüche 1 bis 18,
**dadurch gekennzeichnet, dass** zusätzlich dünne Anpaßschichten und/oder dünne Folien vorgesehen sind.

## Claims

1. Device for treating corporal tissue and/or for disintegrating corporal concretions (4) by means of acoustic energy such as shock, pressure or ultrasonic waves, comprising
- a sound-generating unit (1, 2) for generating acoustic waves;
- if needed, a downstream bundling unit (3) for the acoustic waves; and
- a transmission medium for the acoustic waves generated by the sound-generating unit, for conducting the waves to a body;
**characterized in that** the refractive index of the transmission medium, or the velocity of propagation therein, is matched to surface body tissue, so that no refraction of acoustic waves occurs upon their transition into the body.

2. Device according to claim 1,
**characterized in that** the velocity of propagation of acoustic waves in the transmission medium is in the range of 1350-1400 m/s.

3. Device according to any one of claims 1 or 2,
**characterized in that** the transmission medium is an oil.

4. Device according to claim 3,
**characterized in that** the transmission medium is paraffin oil.

5. Device according to any one of claims 1 to 4,
**characterized in that** a closed receptacle (7) is provided for the transmission medium, the receptacle having a sound-exit surface that is formed by a sound-conducting material (8) and forms an interface or a coupling face.

6. Device according to claim 5,
**characterized in that** the receptacle (7) comprises two chambers separated by a defined sound-transition surface, in an inner chamber of which is provided the sound-generating unit (1, 2) and, if needed, the bundling unit (3) for the acoustic waves, and an outer chamber of which is closed-off from the sound-exit surface (8) forming an interface to the body.

7. Device according to claim 6,
**characterized in that** merely the outer chamber contains a medium having a matched velocity of propagation.

8. Device according to claim 7,
**characterized in that** the inner chamber contains water.

9. Device according to any one of claims 5 to 8,
**characterized in that** the shape of the sound-transition surface can be changed.

10. Device according to any one of claims 1 to 9,
**characterized in that** the sound-exit surface (8) is a diaphragm.

11. Device for treating body tissue according to claim 1,
**characterized in that** an applicator is provided which closes-off a reflector (3) and is designed so that it, when urged under comparatively high pressure against the body, shapes the geometry of the coupling-in face under control, and deforms it, for example to a plane, an aspherical, or a spherical face.

12. Device according to any one of claims 1 to 11,
**characterized in that** additionally an impedance-matching is performed.

13. Device according to any one of claims 1 to 18,
**characterized in that** additionally thin matching-layers and/or thin foils are provided.

## Revendications

1. Dispositif à traiter des tissus corporels et/ou à broyer des calculs corporels (4) moyennant de l'énergie acoustique comme des ondes de choc, des ondes de surpression ou des ondes ultrasonores, comprenant
- une unité génératrice de sons (1, 2), qui engendre les ondes acoustiques,
- éventuellement une unité de focalisation (3) en aval à concentrer les ondes acoustiques, et
- un milieu de transmission à transmettre les ondes acoustiques engendrées par ladite unité génératrice de sons in de les transmettre vers le corps,
**caractérisé en ce que** l'indice de réfraction ou respectivement la vitesse de propagation dudit milieu de transmission est adapté au tissu corporel superficiel d'une telle manière, qu'il y n'ait pas une réfraction des ondes acoustique à leur transfert dans le corps.

2. Dispositif selon la revendication 1,
**caractérisé en ce que** ledit milieu de transmission présente une vitesse de propagation pour ondes acoustiques dans la gamme entre 1350 et 1400 m/s.

3. Dispositif selon une quelconque des revendications 1 ou 2,
**caractérisé en ce que** ledit milieu de transmission est une huile.

4. Dispositif selon la revendication 3,
**caractérisé en ce que** ledit milieu de transmission est une huile de paraffine.

5. Dispositif selon une quelconque des revendications 1 à 4,
**caractérisé en ce qu'**un réservoir fermé (7) est disposé pour ledit milieu de transmission, qui présente une surface de sortie des son formée par un matériau (8) perméable au son, qui constitue la surface limite ou respectivement la surface de couplage.

6. Dispositif selon la revendication 5,
**caractérisé en ce que** ledit réservoir (7) comprend deux chambres séparées par une surface définie de transfert de son, dont lesquelles la chambre intérieure renferme ladite unité génératrice de sons (1, 2) et ladite unité des focalisation (3) éventuellement prévue pour les ondes acoustiques, pendant que la chambre extérieure est isolée de ladite surface de sortie de son (8) constituant la surface limite vers le corps.

7. Dispositif selon la revendication 6,
**caractérisé en ce qu'**un milieu à vitesse de propagation adaptée ne se trouve que dans ladite chambre extérieure.

8. Dispositif selon la revendication 7,
**caractérisé en ce que** de l'eau se trouve dans ladite chambre intérieure.

9. Dispositif selon une quelconque des revendications 5 à 8,
**caractérisé en ce que** la forme de ladite surface de transfert est variable.

10. Dispositif selon une quelconque des revendications 5 à 9,
**caractérisé en ce que** ladite surface de sortie de son (8) est une membrane.

11. Dispositif à traiter du tissu corporel selon la revendication 1,
**caractérisé en ce qu'**un applicateur est disposé, qui ferme ledit réflecteur (3), en étant conçu d'une telle façon, qu'il soit pressé contre le corps à une relativement haute pression, en configurant la géométrie de la surface de couplage dans le corps de manière contrôlée et la déformant, par exemple, en une surface planaire, asphérique ou sphérique.

12. Dispositif selon une quelconque des revendications 1 à 11,
**caractérisé en ce qu'**il y a au plus une adaptation d'impédance.

13. Dispositif selon une quelconque des revendications 1 à 18,
**caractérisé en ce que** des couches adaptatrices minces et/ou des feuilles minces sont disposées au plus.
